# EUROPEAN PATENT APPLICATION

(11) **EP 3 922 350 A1**
(43) Date of publication of application: **15.12.2021**
(21) Application number: 20179173.8
(22) Date of filing: 10.06.2020
(51) Int. Cl.: B01J 21/12, B01J 23/04, B01J 23/10, B01J 23/22, B01J 35/10, C07C 5/333

(54) **SUPPORTED CATALYSTS FOR NON-OXIDATIVE DEHYDROGENATION OF ALKANES**

(71) Applicant: SABIC Global Technologies B.V., 4612 PX Bergen op Zoom (NL)
(72) Inventor: DHACHAPALLY, Naresh, 562125 Bangalore (IN); MELEPPURAM, Nigit J., 562125 Bangalore (IN); DEVASSY, Biju M., 562125 Bangalore (IN); NAIR, Vinod S., 562125 Bangalore (IN)
(74) Representative: J A Kemp LLP

(57) **Abstract**

A supported non-oxidative alkane dehydrogenation catalyst and a method for making and using the same is disclosed. The supported non-oxidative alkane dehydrogenation catalyst can include a vanadium oxide, a rare earth metal oxide, an alkali metal oxide, and a support containing silica and alumina.

## Description

### CROSS REFERENCE TO RELATED APPLICATIONS

None.

### BACKGROUND OF THE INVENTION

### A. Field of the Invention

The invention generally concerns catalysts for non-oxidative dehydrogenation of alkanes. The catalyst can include a vanadium oxide (e.g., V₂O₅), a rare earth metal oxide, and a group 1 metal oxide, supported on a silica and alumina containing support.

### B. Description of Related Art

Alkane dehydrogenation is a recognized process for production of a variety of useful hydrocarbon products, such as isobutylene for conversion to MTBE, isooctane, and alkylates to supplement and enrich gasolines. There are several current catalytic processes useful for catalytic dehydrogenation of light alkanes, including the CATOFIN^{®} process (Houdry, Inc. USA), Oleflex^{®} process (UOP, Inc., USA), and Star process^{®} (ThyssenKrupp Industrial Solutions AG, Germany). The catalysts that are used in these processes are manufactured from two different groups of materials. The CATOFIN^{®} process utilizes chromia-alumina catalysts. In contrast, the catalysts for the Oleflex^{®} process and Star process^{®} utilize a supported precious metal. The CATOFIN^{®} process suffers in that the disposal and/or use of a chromium-based catalyst can pose environmental, health, and economic challenges, while the Oeflex^{®} and Star processes^{®} both use expensive metals.

In an effort to overcome these deficiencies, vanadium-based catalysts for alkane dehydrogenation have been developed. By way of example, U.S. Patent No. 5,220,092 describes a process for producing alkenes by contacting 3 to 12 wt.% vanadia catalyst supported on a gamma alumina support at temperatures of at least 600 °C for less than 4 seconds. The catalyst was made by impregnating pre-calcined alumina with oxalic acid and ammonium polyvanadate and then calcining at 450 °C to produce an alumina supported divanadium pentoxide. While various supported vanadium oxide catalysts are known in the art for the non-oxidative dehydrogenation of alkanes to alkenes, these catalysts suffer from relatively fast deactivation and low catalytic life, decreasing the efficiency and commercial value of the catalysts.

### SUMMARY OF THE INVENTION

A discovery has been made that provides a solution to at least some of the problems associated with supported vanadium-based catalysts. In one aspect, the solution includes a supported non-oxidative alkane dehydrogenation catalyst containing a vanadium oxide, a rare earth metal oxide, and an alkali metal oxide supported on a support containing alumina and silica. As illustrated in a non-limiting manner in the Examples, including rare earth metal oxide and/or silica in the supported vanadium-based catalysts can increase catalytic activity and catalytic life of the catalyst. Without wishing to be bound by theory, lanthanum oxide can increase the dispersion of vanadium oxide in the catalyst by interaction between lanthanum oxide and vanadium oxide. Presence of silica delays the phase transformation of alumina, such as transformation of alumina to inactive alpha-alumina.

One aspect of the present invention is directed to a supported non-oxidative alkane dehydrogenation catalyst. The supported non-oxidative alkane dehydrogenation catalyst can include a vanadium oxide, a rare earth metal oxide, an alkali metal oxide and a support containing alumina and silica. In some particular aspects, the catalyst can include 0.55 to 11 wt. % or 1 to 11 wt. % of vanadium present as an oxide, based on the total weight of the catalyst. In some aspects, the catalyst can include 1 to 20 wt.%, or 2 to 20 wt.%, or 3 to 13 wt.%, or 4 to 8 wt.% of the vanadium oxide represented as V₂O₅, based on the total weight of the catalyst. In some aspects, the vanadium oxide can be V₂O₃, V₂O₄, or V₂O₅, or any combination thereof. In some particular aspects, the vanadium oxide can be V₂O₅. In some aspects, the catalyst can include 0.1 to 3 wt. %, preferably 0.2 to 2 wt. %, more preferably 0.2 to 1 wt. %, of the rare earth metal oxide, such as a lanthanum oxide and/or a cerium oxide, preferably a lanthanum oxide, based on the total weight of the catalyst. In some particular aspects, the catalyst can include 0.08 to 2.6 wt. % or 0.1 to 2.6 wt. % of lanthanum present as an oxide represented as La₂O₃, based on the total weight of the catalyst. In some aspects, the lanthanum oxide can be La₂O₃. In some aspects, the catalyst can include 0.1 to 5 wt.% or 0.1 to 2 wt.% or 0.2 to 1.2 wt.%, of the alkali metal oxide such as a potassium oxide represented as K₂O, based on the total weight of the catalyst. In some particular aspects, the catalyst can include 0.06 to 1.3 wt.% of potassium present as an oxide, based on the total weight of the catalyst. In some aspects, the potassium oxide can be K₂O. In some aspects, the amount of silica present in the support can be 0.1 to 5 wt.% or 0.1 to 2 wt. %, based on the total weight of the catalyst. In some aspects, the amount of alumina present in the support can be 71 to 98.7 wt.% or 80 to 95 wt. %, based on the total weight of the catalyst. In some aspects, the alumina in the support can contain a transition alumina. In some particular aspects, the transition alumina can contain chi (χ) alumina, kappa (κ) alumina, rho (p) alumina, gamma (γ) alumina, eta (η) alumina, delta (δ) alumina, theta (θ) alumina, preferably delta (δ) alumina. In some aspects, the catalyst can include i) 1 to 20 wt.% of a vanadium oxide, ii) 0.1 to 3 wt.% of a rare earth metal oxide, iii) 0.1 to 2 wt.% of an alkali metal oxide, and iv) a support containing 0.1 to 2 wt.% of silica and 71 wt.% to 98.7 wt.% of alumina, where the wt.% is based on the total weight of the catalyst. In some particular aspects, the catalyst can include i) 1 to 20 wt.% of a vanadium oxide represented as V₂O₅, ii) 0.1 to 3 wt.% of a lanthanum oxide, such as La₂O₃, iii) 0.1 to 2 wt.% of a potassium oxide such as K₂O, and iv) a support containing 0.1 to 2 wt.% of silica and 71 wt.% to 98.7 wt.% of delta alumina, where the wt.% is based on the total weight of the catalyst. In some aspects, the vanadium oxide can be V₂O₃, V₂O₄, V₂O₅, or any combination thereof. In some aspects, the vanadium oxide represented as V₂O₅ to the rare earth metal oxide molar ratio in the catalyst can be 0.9:1 to 545:1, 2:1 to 100:1, or 10:1 to 50:1 or 15:1 to 35:1. In some aspects, the vanadium oxide represented as V₂O₅ to the alkali metal oxide molar ratio in the catalyst can be 1.5:1 to 50:1, preferably 2:1 to 25:1, more preferably 2.5:1 to 15:1. In some aspects, the alkali metal oxide to the rare earth metal oxide molar ratio in the catalyst can be 1.1:1 to 100:1, or 1.2:1 to 50:1, or 1.5:1 to 20:1, or 2:1 to 10:1. The catalyst can have a specific surface area of at least 50 m²/g, such as 50 m²/g to 250 m²/g, preferably 80 m²/g to 200 m²/g. The catalyst can have a pore size such as average pore diameter of 30 to 200 Å, preferably 50 to 150 Å.

One aspect of the present invention is directed to a process for making the supported non-oxidative alkane dehydrogenation catalyst. The process can include any one of, any combination of, or all of steps (a), (b), and (c). In step (a) a calcined support containing alumina and silica can be impregnated with a vanadium precursor, a rare earth metal precursor, and a alkali metal precursor to obtain an impregnated support. In step (b) the impregnated support can be dried to obtain a dried impregnated support. In step (c) the dried impregnated support can be calcined to obtain the supported non-oxidative alkane dehydrogenation catalyst. In some aspects, in step (a) the calcined support can be contacted with solution(s) such as aqueous solution(s) containing the vanadium precursor, the rare earth metal precursor, and/or the alkali metal precursor. In some particular aspects, in step (a) the calcined support can be impregnated with a precursor solution such as an aqueous solution containing the vanadium oxide precursor, the rare earth metal oxide precursor and the alkali metal oxide precursor, by an incipient wet impregnation method. In some aspects, drying in step (b) can include heating the impregnated support at a temperature 50 °C to 180 °C. In some aspects, calcining in step (c) can include heating the dried impregnated support at a temperature 500 to 1000 °C or 500 to 850 °C or 600 °C to 1000 °C or 700 °C to 900 °C or 700 °C to 800 °C in the presence of air and/or O₂.

In some aspects, the process can further include making the calcined support and can further include any one of, any combination of, or all of steps (d), (e), (f) and/or (g). In step (d) an alumina precursor can be contacted with a silica precursor under conditions suitable to obtain a shapeable support precursor. In step (e) the shapeable support precursor can be shaped to obtain a wet shaped support precursor. In step (f) the wet shaped support precursor can be dried to obtain a dry shaped support precursor. In step (g) the dry shaped support precursor can be calcined to obtain the calcined support. In some aspects, in step (d) the alumina precursor can be contacted with a solution such as a colloidal solution containing the silica precursor. In some aspects, the colloidal solution can contain a peptizing agent such as nitric acid and/or acetic acid. In some aspects, shaping in step (e) can include extruding the shapeable support precursor. In some aspects, drying in step (f) can include heating the wet shaped support precursor at a temperature 50 °C to 180 °C. In some aspects, calcining in step (g) can include heating the dry shaped support precursor at a temperature 600 °C to 1000 °C or 700 °C to 900 °C or 700 °C to 800 °C to in presence of air and/or O₂.

Another aspect of the present invention is directed to a process for producing an alkene. The process can include contacting a feed stream containing an alkane with a supported non-oxidative alkane dehydrogenation catalyst under conditions sufficient to non-oxidatively dehydrogenate at least a portion of the alkane and produce a products stream containing an alkene. The contacting conditions can include a temperature of 400 °C to 800 °C, a pressure of 0.01 MPa to 0.5 MPa, or a GHSV of the feed stream of 0.5 L g⁻¹ h⁻¹ to 10 L g⁻¹ h⁻¹, or any combination thereof. Oxygen (e.g., O₂ gas) does not have to be used in this process. In some aspects, the alkane can be one or more of a C₂ to C₁₀ alkane, preferably one or more of a C₂ to C₄ alkane. In some particular aspects, the alkane can be iso-butane and the alkene can be iso-butylene. In some aspects, prior to contacting with the feed stream, the catalyst can be activated to form an activated catalyst and the activated catalyst can be contacted with the feed stream. In some particular aspects, the activation process can include contacting the catalyst with an oxidizing source at a temperature 400 °C to 800 °C to form an oxidized catalyst, and contacting the oxidized catalyst with a reducing source at a temperature 400 °C to 800 °C to form the activated catalyst.

The following includes definitions of various terms and phrases used throughout this specification.

An "alkane" is a linear or branched, substituted or unsubstituted, saturated hydrocarbon. Non-limiting examples of alkane substituents include alkyl, aryl, hydroxyl, alkyloxy, carboxylic acid, ester, amine, amide, nitrile, acyl, thiol, and thioether.

An "aryl" group or an "aromatic" group is a substituted or unsubstituted, mono- or polycyclic hydrocarbon with alternating single and double bonds within each ring structure. Non-limiting examples of aryl group substituents include alkyl, hydroxyl, alkyloxy, carboxylic acid, ester, amine, amide, nitrile, acyl, thiol, and thioether.

An "alkene" is linear or branched, substituted or unsubstituted, unsaturated hydrocarbon. Alkenes include one or more degree of unsaturation. Non-limiting examples of alkene substituents include alkyl, aryl, hydroxyl, alkyloxy, carboxylic acid, ester, amine, amide, nitrile, acyl, thiol, and thioether.

Rare earth metals include the fifteen lanthanides on the IUPAC Periodic Table of the Elements, as well as scandium and yttrium.

The terms "about" or "approximately" are defined as being close to as understood by one of ordinary skill in the art. In one non-limiting embodiment, the terms are defined to be within 10%, preferably within 5%, more preferably within 1%, and most preferably within 0.5%.

The terms "wt.%," "vol.%," or "mol.%" refer to a weight percentage of a component, a volume percentage of a component, or molar percentage of a component, respectively, based on the total weight, the total volume of material, or total moles, that includes the component. In a non-limiting example, 10 grams of component in 100 grams of the material is 10 wt.% of component.

The term "substantially" and its variations are defined to include ranges within 10%, within 5%, within 1%, or within 0.5%.

The terms "inhibiting" or "reducing" or "preventing" or "avoiding" or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the words "a" or "an" when used in conjunction with any of the terms "comprising," "including," "containing," or "having" in the claims, or the specification, may mean "one," but it is also consistent with the meaning of "one or more," "at least one," and "one or more than one."

The phrase "and/or" can include "and" or "or." To illustrate, A, B, and/or C can include: A alone, B alone, C alone, a combination of A and B, a combination of A and C, a combination of B and C, or a combination of A, B, and C.

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The catalysts and processes of the present invention can "comprise," "consist essentially of," or "consist of" particular ingredients, components, compositions, steps, *etc.* disclosed throughout the specification. With respect to the transitional phrase "consisting essentially of," in one non-limiting aspect, a basic and novel characteristic of the catalysts of the present invention are their abilities to catalyze non-oxidative dehydrogenation of alkanes.

Other objects, features, and advantages of the present invention will become apparent from the following figures, detailed description, and examples. It should be understood, however, that the figures, detailed description, and examples, while indicating specific embodiments of the invention, are given by way of illustration only and are not meant to be limiting. Additionally, it is contemplated that changes and modifications within the spirit and scope of the invention will become apparent to those skilled in the art from this detailed description. Other embodiments of the invention are discussed throughout this application. Any embodiment discussed with respect to one aspect of the invention applies to other aspects of the invention as well and vice versa. Each embodiment described herein is understood to be embodiments of the invention that are applicable to other aspects of the invention. It is contemplated that any embodiment discussed herein can be implemented with respect to any method or composition of the invention, and vice versa. Furthermore, compositions of the invention can be used to achieve methods of the invention.

### BRIEF DESCRIPTION OF THE DRAWINGS

Advantages of the present invention may become apparent to those skilled in the art with the benefit of the following detailed description and upon reference to the accompanying drawings.

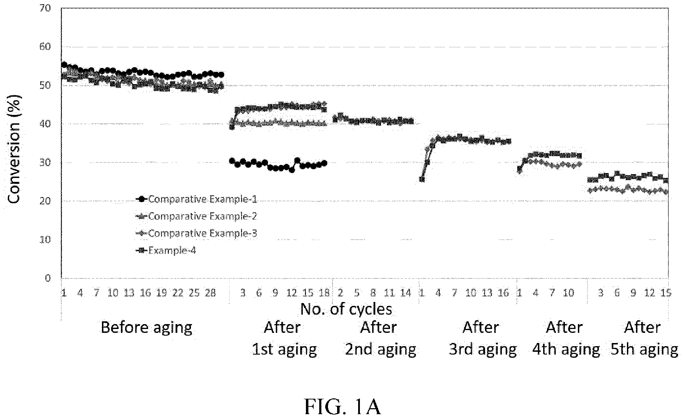

**FIG. 1A** shows isobutane conversion for the comparative catalysts (Examples 1, 2, and 3) and a catalyst (Example 4) according to an example present invention, before and after aging.

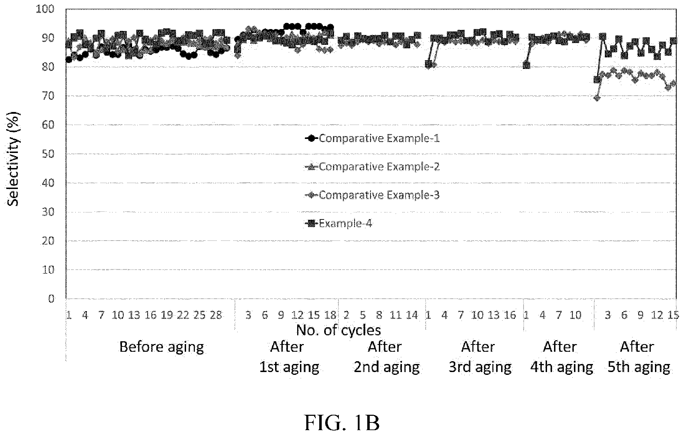

**FIG. 1B** shows isobutylene selectivity for the comparative catalysts (Examples 1, 2, and 3) and a catalyst (Example 4) according to an example present invention, before and after aging.

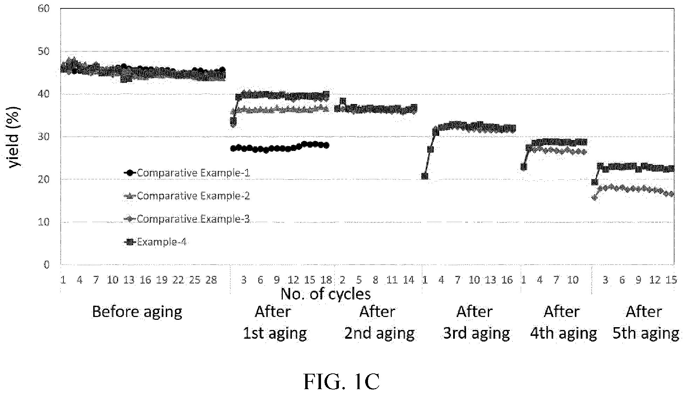

**FIG. 1C** shows isobutane yield for the comparative catalysts (Examples 1, 2, and 3) and a catalyst (Example 4) according to an example present invention, before and after aging.

While the invention is susceptible to various modifications and alternative forms, specific embodiments thereof are shown by way of example in the drawings. The drawings may not be to scale.

### DETAILED DESCRIPTION OF THE INVENTION

A discovery has been made that provides a solution to at least some of the problems associated with supported vanadium catalysts used in non-oxidative dehydrogenation of alkanes to produce alkenes. As illustrated in a non-limiting manner in the Examples, the supported non-oxidative alkane dehydrogenation catalysts of the present invention have relatively less deactivation with aging.

These and other non-limiting aspects of the present invention are discussed in further detail in the following sections.

### A. Supported Non-oxidative Alkane Dehydrogenation catalyst

The supported non-oxidative alkane dehydrogenation catalyst can include a vanadium oxide, a rare earth metal oxide, an alkali metal oxide supported on a support containing alumina and silica. In some aspects, the catalyst can include 1 to 20 wt.%, or at least any one of, equal to any one of, or between any two of 1 wt.%, 2 wt.%, 3 wt.%, 4 wt.%, 5 wt.%, 6 wt.%, 7 wt.%, 8 wt.%, 9 wt.%, 10 wt.%, 11 wt.%, 12 wt.%, 13 wt.%, 14 wt.%, 15 wt.%, 16 wt.%, 17 wt.%, 18 wt.%, 19 wt.%, and 20 wt.% of a vanadium oxide, represented as V₂O₅. In some aspects, the catalyst can include one or more vanadium oxides. The vanadium oxide can include VO, V₂O₃, V₂O₄, or V₂O₅, or vanadate ions or any combination thereof. Non-limiting examples of vanadate ions can include VO₄³⁻, V₂O₇⁴⁻, V₃O₉³⁻, V₄O₁₂⁴⁻, V₅O₁₄³⁻, and the like. In some aspects, the vanadium oxide can include distinct phases of the vanadium oxides such as VₙO₂ₙ₊₁, VₙO₂ₙ₋₁, and non-stoichimetric vanadium oxygen phases. Phases with the general formula VₙO₂ₙ₊₁, wherein n is a whole number greater than zero exist between V₂O₅ (vanadium (V) species) and VO₂ (vanadium (IV) species). Examples of these phases include V₃O₇, V₄O₉ and V₆O₁₃. Phases with the general formula VₙO₂ₙ₋₁, wherein n is a whole number greater than zero exist between VO₂ (vanadium (IV) species) and V₂O₃ (vanadium (III) species). Examples of these phases include V₄O₇, V₅O₉, V₆O₁₁, V₇O₁₃ and V₈O₁₅. In some particular aspects, the vanadium oxide is V₂O₅. The alkali metal can be lithium, sodium, potassium, rubidium, or cesium or any combination thereof. In some particular aspect, the alkali metal can be potassium. In some aspects, the catalyst can include 0.1 to 5 wt.% or at least any one of, equal to any one of, or between any two of 0.1 wt.%, 0.2 wt.%, 0.3 wt.%, 0.4 wt.%, 0.5 wt.%, 0.6 wt.%, 0.7 wt.%, 0.8 wt.%, 0.9 wt.%, 1.0 wt.%, 1.2 wt.%, 1.3 wt.%, 1.4 wt.%, 1.5 wt.%, 1.6 wt.%, 1.7 wt.%, 1.8 wt.%, 1.9 wt.%, 2 wt.%, 2.1 wt.%, 2.2 wt.%, 2.3 wt.%, 2.4 wt.%, 2.5 wt.%, 2.6 wt.%, 2.7 wt.%, 2.8 wt.%, 2.9 wt.%, 3.0 wt.%, 3.1 wt.%, 3.2 wt.%, 3.3 wt.%, 3.4 wt.%, 3.5 wt.%, 3.6 wt.%, 3.7 wt.%, 3.8 wt.%, 3.9 wt.%, 4.0 wt.%, 4.1 wt.%, 4.2 wt.%, 4.3 wt.%, 4.4 wt.%, 4.5 wt.%, 4.6 wt.%, 4.7 wt.%, 4.8 wt.%, 4.9 wt.%, and 5.0 wt.% of a alkali metal oxide, such as potassium oxide. In some aspects, the potassium oxide can be K₂O. The rare earth metal can be lanthanum, cerium, neodymium, samarium, or any combination thereof, preferably lanthanum and/or cerium, more preferably lanthanum. In some aspects, the catalyst can include 0.1 to 3 wt.% or at least any one of, equal to any one of, or between any two of 0.1 wt.%, 0.2 wt.%, 0.3 wt.%, 0.4 wt.%, 0.5 wt.%, 0.6 wt.%, 0.7 wt.%, 0.8 wt.%, 0.9 wt.%, 1.0 wt.% 1.1 wt.%, 1.2 wt.%, 1.5 wt.%, 2.0 wt.%, 2.5 wt.%, and 3.0 wt.%, of the rare earth metal oxide, such as a lanthanum oxide and/or cerium oxide, preferably a lanthanum oxide. In some aspects, the lanthanum oxide can be La₂O₃. In some aspects, the catalyst can include 0.1 to 2 wt.% or at least any one of, equal to any one of, or between any two of 0.1 wt.%, 0.2 wt.%, 0.3 wt.%, 0.4 wt.%, 0.5 wt.%, 0.6 wt.%, 0.7 wt.%, 0.8 wt.%, 0.9 wt.%, 1.0 wt.%, 1.2 wt.%, 1.3 wt.%, 1.4 wt.%, 1.5 wt.%, 1.6 wt.%, 1.7 wt.%, 1.8 wt.%, 1.9 wt.%, and 2 wt.% of silica in the support based on total weight of the catalyst. In some aspects, the catalyst can include 80 to 95 wt.% or at least any one of, equal to any one of, or between any two of 80 wt. %, 81 wt.%, 82 wt.%, 83 wt.%, 84 wt.%, 85 wt.%, 86 wt.%, 87 wt.%, 88 wt.%, 89 wt.%, 90 wt.%, 91 wt.%, 92 wt.%, 93 wt.%, 94 wt.%, and 95 wt.%, of alumina in the support based on total weight of the catalyst. In some aspects, the alumina can contain a transition alumina. The transition alumina can contain chi alumina, kappa alumina, rho alumina, gamma alumina, eta alumina, delta alumina, theta alumina or any combination thereof. In some particular aspect, the transition alumina can contain delta alumina. In some particular aspects, the catalyst can include i) 1 to 20 wt.%, or at least any one of, equal to any one of, or between any two of 1 wt.%, 2 wt.%, 3 wt.%, 4 wt.%, 5 wt.%, 6 wt.%, 7 wt.%, 8 wt.%, 9 wt.%, 10 wt.%, 11 wt.%, 12 wt.%, 13 wt.%, 14 wt.%, 15 wt.%, 16 wt.%, 17 wt.%, 18 wt.%, 19 wt.%, and 20 wt.% of a vanadium oxide represented as V₂O₅, ii) 0.1 to 3 wt.% or at least any one of, equal to any one of, or between any two of 0.1 wt.%, 0.2 wt.%, 0.3 wt.%, 0.4 wt.%, 0.5 wt.%, 0.6 wt.%, 0.7 wt.%, 0.8 wt.%, 0.9 wt.%, 1.0 wt.% 1.1 wt.%, 1.2 wt.%, 1.5 wt.%, 2.0 wt.%, 2.5 wt.%, and 3.0 wt.%, of a lanthanum oxide such as La₂O₃, and iii) 0.1 to 2 wt.% or at least any one of, equal to any one of, or between any two of 0.1 wt.%, 0.2 wt.%, 0.3 wt.%, 0.4 wt.%, 0.5 wt.%, 0.6 wt.%, 0.7 wt.%, 0.8 wt.%, 0.9 wt.%, 1.0 wt.%, 1.2 wt.%, 1.3 wt.%, 1.4 wt.%, 1.5 wt.%, 1.6 wt.%, 1.7 wt.%, 1.8 wt.%, 1.9 wt.%, and 2 wt.% of a potassium oxide such as K₂O, and iv) a support containing 0.1 to 5 wt.% or at least any one of, equal to any one of, or between any two of 0.1 wt.%, 0.2 wt.%, 0.3 wt.%, 0.4 wt.%, 0.5 wt.%, 0.6 wt.%, 0.7 wt.%, 0.8 wt.%, 0.9 wt.%, 1.0 wt.%, 1.2 wt.%, 1.3 wt.%, 1.4 wt.%, 1.5 wt.%, 1.6 wt.%, 1.7 wt.%, 1.8 wt.%, 1.9 wt.%, 2 wt.%, 2.1 wt.%, 2.2 wt.%, 2.3 wt.%, 2.4 wt.%, 2.5 wt.%, 2.6 wt.%, 2.7 wt.%, 2.8 wt.%, 2.9 wt.%, 3.0 wt.%, 3.1 wt.%, 3.2 wt.%, 3.3 wt.%, 3.4 wt.%, 3.5 wt.%, 3.6 wt.%, 3.7 wt.%, 3.8 wt.%, 3.9 wt.%, 4.0 wt.%, 4.1 wt.%, 4.2 wt.%, 4.3 wt.%, 4.4 wt.%, 4.5 wt.%, 4.6 wt.%, 4.7 wt.%, 4.8 wt.%, 4.9 wt.%, and 5.0 wt.% of silica and 71 to 98.7 wt.% or at least any one of, equal to any one of, or between any two of 71 wt.%, 72 wt.%, 73 wt.%, 74 wt.%, 75 wt.%, 76 wt.%, 77, wt.%, 78 wt.%, 79 wt.%, 80 wt. %, 81 wt.%, 82 wt.%, 83 wt.%, 84 wt.%, 85 wt.%, 86 wt.%, 87 wt.%, 88 wt.%, 89 wt.%, 90 wt.%, 91 wt.%, 92 wt.%, 93 wt.%, 94 wt.%, 95 wt.%, 96 wt.%, 97 wt.%, 98 wt.%, and 98.7 wt.% of delta alumina, where the wt. % is based on the total weight of the catalyst. In some aspects, the catalyst can have a specific surface area of at least 50 m²/g, such as 50 m²/g to 250 m²/g or at least any one of, equal to any one of, or between any two of 50, 60, 70, 80, 85, 90, 95, 100, 105, 110, 115, 120, 125, 130, 135, 140, 145, 150, 155, 160, 165, 170, 175, 180, 185, 190, 195, 200, 210, 220, 230, 240, and 250 m²/g. In some aspects, the catalyst can have an average pore diameter of 30 Å to 200 Å, or at least any one of, equal to any one of, or between any two of 30, 40, 50, 55, 60, 65, 70, 75, 80, 85, 90, 95, 100, 105, 110, 115, 120, 125, 130, 135, 140, 145, 150, 160, 170, 180, 190, and 200 Å. In some aspects, the catalyst does not include chromium and does not include gold, platinum or palladium. In some instances, the support for the catalysts of the present invention consists of or consists essentially of silica and alumina.

### B. Process for Producing the Supported Non-oxidative Alkane Dehydrogenation Catalyst

The process can include the steps of impregnating a calcined support containing silica and alumina, with a vanadium precursor, a rare earth metal precursor, and an alkali metal precursor to form an impregnated support, drying the impregnated support to obtain a dried impregnated support, and calcining the dried impregnated support to obtain the supported non-oxidative alkane dehydrogenation catalyst. In some aspects, the calcined support prior to impregnation can be dried (e.g., heat treated) in presence of an oxidizing source (e.g., air, oxygen, or oxygen enriched air) at 70 °C to 150 °C, or 115 °C to 125 °C, or any range or value there between for a desired amount of time (e.g., 1 to 24 hours). The calcined support can have a specific surface area of 80 to 200 m²/g, or at least any one of, equal to any one of, or between any two of 80, 90, 100, 120, 125, 130, 135, 140, 145, 150, 155, 160, 165, 170, 175, 180, 185, 190, 195, and 200 m²/g. In some particular aspects, the alumina in the calcined support can contain delta alumina.

The impregnation method can be wet impregnation method or incipient wet impregnation method. In some aspects, the calcined support can be impregnated with a precursor solution containing the vanadium precursor, the rare earth metal precursor and the alkali metal precursor using wet incipient impregnation method. The precursor solution can be an aqueous solution containing the vanadium precursor, the rare earth metal precursor, and the alkali metal precursor. By way of example, the precursor solution can be prepared by dissolving the vanadium precursor, the rare earth metal precursor, and the alkali metal precursor in water. In some aspects, the vanadium precursor can be vanadyl oxalate (VOC₂O₄), ammonium metavanadate, ammonium vanadium oxalate, vanadium monocarboxylate, vanadium dicarboxylate, or vanadium tricarboxylate or any combination thereof. In some aspects, the rare earth metal precursor can be a lanthanum precursor and/or a cerium precursor, preferably a lanthanum precursor. In some particular aspects, the lanthanum precursor can be nitrate, sulfate and/or halide salt(s) of lanthanum (III). In some particular aspects, the lanthanum precursor can be lanthanum (III) nitrate hexahydrate. In some particular aspects, the cerium precursor can be nitrate, sulfate and/or halide salt(s) of cerium (III). In some aspects, the alkali metal precursor can be a potassium precursor. In some aspects, the potassium precursor can be potassium nitrate, potassium acetate (KC₃H₃O₂), potassium citrate (K₃C₆H₅O₇), potassium oxalate (C₂K₂O₄), or potassium carbonate (K₂CO₃) or any combination thereof. In some particular aspects, the calcined support can be impregnated with a precursor solution containing a vanadium precursor such as vanadyl oxalate, a lanthanum precursor such as lanthanum (III) nitrate hexahydrate and a potassium precursor such as potassium nitrate using wet incipient impregnation method. The amount of vanadium precursor, rare earth metal precursor and alkali metal precursor to be used can be calculated based on the weight percents of vanadium, the rare earth metal and the alkali metal, respectively, to be obtained in the final catalyst. In some aspects, the impregnation can be performed at 20 to 35 °C (e.g., room temperature) for a desired amount of time (e.g., at least 0.5 hours or 1 to 24 hours, 1 to 20 hours, 1 to 15 hours, preferably 12 hours).

In some aspects, the impregnated support can be dried at 70 °C to 180 °C or at least any one of, equal to any one of, or between any two of 70, 80, 90, 100, 110, 120, 130, 140, 150, 160, 170 and 180 °C for 1 to 24 hours, or at least any one of, equal to any one of, or between any two of 1, 2, 4, 6, 8, 10, 12, 14, 16, 18, 20, 22 and 24 hours to obtain the dry impregnated support. In some particular aspects, the impregnated support can be dried at about 120 °C for about 16 hours to obtain the dry impregnated support. The dry impregnated support can be calcined in the presence of an oxidizing source, such as air, O₂ enriched air, and/or O₂ at a temperature of 500 °C to 1000 °C, or 600 °C to 1000 °C, or at least, equal to, or between any two of 500, 600, 650, 700, 750, 800, 850, 900, 950, and 1000 °C to obtain the supported non-oxidative alkane dehydrogenation catalyst.

In some aspects, the process can further include preparing the calcined support. The calcined support can be prepared by contacting an alumina precursor with a silica precursor to obtain a shapeable support precursor, shaping the shapeable support precursor to obtain a wet shaped support precursor, drying the wet shaped support precursor to obtain a dry shaped support precursor and calcining the dry shaped support precursor to obtain the calcined support.

In some aspects, the alumina precursor can be contacted with a solution such as a colloidal solution containing the silica precursor to obtain the shapeable support precursor. In some aspects, the alumina precursor can be aluminum hydroxide. In some aspects, the silica containing solution can contain a peptizing agent. In some aspects, the peptizing agent can be an acid such as nitric acid and/or acetic acid. In some aspects, the aluminum hydroxide can be gibbsite, bayerite, nordstrandite, boehmite, diaspore, tohdite, amorphous aluminum hydroxide, or any combination thereof. In some particular aspects, the aluminum hydroxide can be boehmite. In some aspects, the silica precursor can be a colloidal silica (e.g. LUDOX^{®} AS-40 colloidal silica commercially available from Sigma-Aldrich). The shapeable support precursor of the current invention can be a sufficiently pliable semisolid mass, such as a dough that can be shaped with a shaping process to form the wet shaped support precursor having a desired geometric shape. In some aspects, the shaping process can be an extrusion process and the shapeable support precursor can be extruded (i.e., pushed and/or drawn through an extrusion die and/or an orifice) to form the wet shaped support precursor having a desired shape or configuration. The extrusion of the shapeable support precursor can be performed with any suitable extruder and/or suitable extrusion die and/or orifice, as will be appreciated by those of skill. The die opening and the cross-section of the wet shaped support precursor can have any suitable regular and/or irregular shape. Non-limiting shapes include circular, oval, square, rectangular, pentagonal, hexagonal, rounded square, rounded rectangular, rounded pentagonal, rounded hexagonal, and star shaped. The extrusion die can have one or more opening(s). The extrusion process can be carried out using a ram extruder, a single screw extruder, or a twin screw extruder. In some particular aspects, the extrusion die can have circular opening with 0.5, 1, 2, 3, 4, 5, 6, 7, 8, 9 to 10 mm diameter.

In some aspects, the wet shaped support precursor can be dried at 70 °C to 180 °C or at least any one of, equal to any one of, or between any two of 70, 80, 90, 100, 110, 120, 130, 140 150, 160, 170, and 180 °C for 1 to 24 hours, or at least any one of, equal to any one of, or between any two of 1, 2, 4, 6, 8, 10, 12, 14, 16, 18, 20, 22 and 24 hours to obtain the dry shaped support precursor. In some particular aspects, the wet shaped support precursor can be dried at about 120 °C for about 16 hours to obtain the dry shaped support precursor. The dry shaped support precursor can be calcined in the presence of an oxidizing source, such as air, O₂ enriched air, and/or O₂ at a temperature of 500 °C to 1000 °C or 600 °C to 1000 °C, or at least, equal to, or between any two of 500, 600, 650, 700, 750, 800, 850, 900, 950, and 1000 °C to obtain the calcined support.

The calcined support made through the shaping process such as the extrusion process can contain macro-sized particles. In some aspects, the macro-sized particles can have at least one dimension such as length, width, height, diameter greater than 0.3 mm. The calcined support (e.g., the macro-sized particles of the calcined support) can have a desired geometric shape. The desired geometric shape includes, but is not limited to, spherical, cube, cuboidal, cylindrical, puck, oval, buckyball, and oblong shapes. Macro-sized particles having other shapes can also be made. In some aspects, the macro-sized particles can have cylindrical shape with a circular, elliptical, ovular, triangular, square, rectangular, pentagonal, or hexagonal cross section, although cylindrical shaped macro-sized particles having a cross-section of other shapes can also be made.

### C. Process for Non-oxidative Dehydrogenation of Alkanes

The supported non-oxidative dehydrogenation catalyst of the present invention can catalyze non-oxidative dehydrogenation of an alkane to produce an alkene. In some aspects, a feed stream containing an alkane can be contacted with the supported non-oxidative alkane dehydrogenation catalyst under conditions sufficient to non-oxidatively dehydrogenate at least a portion of the alkane to produce a products stream containing an alkene. The supported non-oxidative dehydrogenation catalyst can be contacted with the feed stream in a dehydrogenation reactor. In some aspects, the dehydrogenation reactor can be a fixed bed reactor. In some aspects, supported non-oxidative dehydrogenation catalyst can be included in the reactor bed of the fixed bed dehydrogenation reactor and during the non-oxidative dehydrogenation of alkane, the feed stream can be passed through and/or over the reactor bed. In some aspects, the contacting condition (e.g., the reactor condition during non-oxidative dehydrogenation of alkane) can include i) a temperature of 400 °C to 800 °C, or 500 °C to 700 °C, or at least any one of, equal to any one of, or between any two of 400, 450, 500, 550, 600, 650, 700, 750, and 800 °C, ii) a gas hourly space velocity (GHSV) of the feed stream of at least 0.1 L h⁻¹ g⁻¹ or 0.5 to 10 L h⁻¹ g⁻¹, or at least any one of, equal to any one of, or between any two of 0.1, 0.5, 1, 2, 3, 4, 5, 6, 7, 8, 9 and 10 L h⁻¹ g⁻¹ or iii) a pressure of 0.01 MPa to 0.5 MPa, or at least any one of, equal to any one of, or between any two of 0.01, 0.02, 0.05, 0.1, 0.2, 0.3, 0.4, and 0.5 MPa, or any combination thereof. The conditions for alkene production can be varied based on the reactor type and/or properties. In some aspects, the feed stream can include at least 50 vol.%, or 82 vol.% to 95 vol.%, or 88 vol.% to 92 vol.% at least any one of, equal to any one of, or between any two of 50 vol.%, 51 vol.%, 52 vol.%, 53 vol.%, 54 vol.%, 55 vol.%, 56 vol.%, 57 vol.%, 58 vol.%, 59 vol.%, 60 vol.%, 61 vol.%, 62 vol.%, 63 vol.%, 64 vol.%, 65 vol.%, 66 vol.%, 67 vol.%, 68 vol.%, 69 vol.%, 70 vol.%, 71 vol.%, 72 vol.%, 73 vol.%, 74 vol.%, 75 vol.%, 76 vol.%, 77 vol.%, 78 vol.%, 79 vol.%, 80 vol.%, 81 vol.%, 82 vol.%, 83 vol.%, 84 vol.%, 85 vol.%, 86 vol.%, 87 vol.%, 88 vol.%, 89 vol.%, 90 vol.%, 91 vol.%, 92 vol.%, 93 vol.%, 94 vol.%, and 95 vol.%, of the alkane. The feed stream (e.g., alkanes in the feed stream) can be obtained from other process units and/or from commercial sources.

The feed stream can contain one or more of a C₂ to C₁₀ alkane and correspondingly the products stream can contain one or more of a C₂ to C₁₀ alkene. C₂ to C₁₀ alkane refers to an alkane having from 2 to 10 carbon atoms, (e.g., including but not limited to ethane, propane, n-butane, iso-butane, n-pentane, iso-pentane, hexane, heptane, octane, nonane, decane). C₂ to C₁₀ alkene refers to an alkene having from 2 to 10 carbon atoms, (e.g., including but not limited to ethylene, propylene, n-butylene, iso-butylene, n-pentene, iso-pentene, hexene, heptene, octene, nonene, decene). Non-limiting examples of alkanes in the feed stream can include ethane, propane, n-butane, isobutane, n-pentane, isopentane, hexane, heptane, octane, nonane, decane, and all isomers thereof. In some particular aspects, the feed stream can contain ethane and the products stream can contain ethylene. In some particular aspects, the feed stream can contain propane and the products stream can contain propylene. In some particular aspects, the feed stream can contain n-butane and the products stream can contain n-butylene. In some particular aspects, the feed stream can contain iso-butane and the products stream can contain iso-butylene. In some particular aspects, the feed stream can contain n-pentane and the products stream can contain n-pentene. In some particular aspects, the feed stream can contain iso-pentane and the products stream can contain iso-pentene. In some particular aspects, the feed stream can contain ethane, propane, n-butane, iso-butane, n-pentane, or iso-pentane or any combination thereof, and correspondingly the products stream can contain ethylene, propylene, n-butylene, iso-butylene, n-pentene or iso-pentene or any combination thereof.

Unlike oxidative dehydrogenation of alkanes, oxygen such as gas phase oxygen and/or lattice oxygen is not used during non-oxidative dehydrogenation of alkanes. In some aspects, the non-oxidative alkane dehydrogenation catalyst can be activated prior to contacting the catalyst with the feed stream and the feed stream can be contacted with the activated catalyst. In some particular aspects, the activation process can include contacting the catalyst in the dehydrogenation reactor with an oxidizing stream (e.g., a gaseous stream containing air, O₂, and/or O₂ enriched air) at a temperature 400 to 1000 °C for a desired amount of time (e.g., 0.01 hr to 1 hr) to form an oxidized catalyst, and contacting the oxidized catalyst with a reducing stream (e.g., a gaseous stream containing H₂) at 400 °C to 800 °C for a desired amount of time (e.g., 0.01 hr to 1 hr) to form the activated catalyst. In some aspects, an inert gas can be passed through the dehydrogenation reactor containing the oxidized catalyst prior to contacting the oxidized catalyst with the reducing stream. In some aspects, an inert gas can be passed through the dehydrogenation reactor containing the activated catalyst prior to contacting the activated catalyst with the feed stream. In some aspects, the inert gas can be nitrogen (N₂) and/or helium. In some aspects, a deactivated catalyst can be formed from the activated catalyst during and/or after the non-oxidative dehydrogenation of alkane. In some aspects, the deactivated catalyst can be activated using the activation process and the process for non-oxidative dehydrogenation of alkane can be repeated. In some aspects, the products stream can contain unreacted alkanes. In some aspects, at least a portion of the unreacted alkanes can be recycled and included in the feed stream to maximize the overall conversion of alkane to alkene via dehydrogenation. In some aspects, the alkenes produced can be further purified, stored and/or used in various processes.

The dehydrogenation reactor can include one or more heating and/or cooling devices (e.g., insulation, electrical heaters, jacketed heat exchangers in the wall) or controllers (e.g., computers, flow valves, automated values, etc.) that are used to control the reactant flow, reaction temperature, and/or pressure of the reaction mixture. While only one reactor is shown, it should be understood that multiple reactors can be housed in one unit or a plurality of reactors housed in one heat transfer unit.

### EXAMPLES

The present invention will be described in greater detail by way of specific examples. The following examples are offered for illustrative purposes only, and are not intended to limit the invention in any manner. Those of skill in the art will readily recognize a variety of noncritical parameters which can be changed or modified to yield essentially the same results.

### Comparative Example 1

### (Synthesis of Vanadium Oxide/K₂O Supported on Delta Alumina Catalyst)

Alumina extrudates mainly in delta-alumina (δ-Al₂O₃) form were prepared using following procedure. Boehmite (G-250) material was extrudated using acetic acid as a peptizing agent. To boehmite powder (500 g) was added dropwise 5% acetic acid (370 ml) and the mixture was mixed for about 30 minutes. The obtained dough was then extruded using a lab extruder (Sunsai), the dies being 3.5 mm in diameter. The prepared wet extrudates were dried at 120 °C for 16 hours in air oven. The dried extrudates (160 g) were calcined at 900 °C for 10 hours in muffle furnace with heating rate 5 °C/min and air flow rate being 480±10 ml/min. The obtained extrudates (size: 3 mm diameter and 6-8 mm length) with surface area of 145 m²/g were used for catalyst preparation. The alumina extrudate support (about 100 g) was heat treated in an oven at 120 °C for 16 hours in the presence of air to remove moisture. The dried alumina extrudate support, after cooling to room temperature, was used for catalyst preparation by incipient wetness impregnation method. The catalyst was prepared by incipient wetness impregnation of the support with an aqueous solution of vanadyl oxalate, VOC₂O₄2H₂O (3.97 g) and potassium nitrate (0.386 g). The impregnation was carried out by contacting the impregnation solution (18 mL) with alumina extrudate support (27.93 g) at room temperature. The impregnated alumina support was then kept at room temperature for 12 hours and then dried at 120 °C for 16 hours. The dried sample was then calcined at 750 °C for 4 hours with heating rate 5 °C/min in the presence of air (flow rate, 8 ml g⁻¹min⁻¹) in a down flow tubular furnace. After calcination, the catalyst was cooled in presence of air and the catalyst was stored in an air tight container. The final calculated composition of the catalyst corresponds to V₂O₅ - 6.3 wt. %, K₂O - 0.6 wt. % with remaining part comprising δ-Al₂O₃.

### Comparative Example 2

### (Synthesis of Vanadium Oxide - Lanthanum oxide - K₂O Supported Delta Alumina)

Alumina extrudates mainly in delta-alumina (δ-Al2O3) form were prepared using following procedure. Boehmite (G-250) material was extrudated using acetic acid as a peptizing agent. To boehmite powder (500 g) was added dropwise 5% acetic acid (370 ml) and the mixture was mixed for about 30 minutes. The obtained dough was then extruded using a lab extruder (Sunsai), the dies being 3.5 mm in diameter. The prepared wet extrudates were dried at 120 °C for 16 hours in air oven. The dried extrudates (160 g) were calcined at 900 °C for 10 hours in muffle furnace with heating rate 5 °C/min and air flow rate being 480±10 ml/min. The obtained extrudates (size: 3 mm diameter and 6-8 mm length) with surface area of 145 m²/g were used for catalyst preparation. The alumina extrudate support (about 100 g) was heat treated in an oven at 120 °C for 16 hours in the presence of air to remove moisture. The dried alumina extrudate support, after cooling to room temperature, was used for catalyst preparation by incipient wetness impregnation method. The catalyst was prepared by incipient wetness impregnation of the support with an aqueous solution of vanadyl oxalate, VOC₂O₄2H₂O (3.97 g), potassium nitrate (0.386 g) and lanthanum nitrate (0.399 g). The impregnation was carried out by contacting the impregnation solution (18 mL) with alumina extrudate support (27.93 g) at room temperature. The impregnated alumina support was then kept at room temperature for 12 hours and then dried at 120 °C for 16 hours. The dried sample was then calcined at 750 °C for 4 hours with heating rate 5 °C/min in the presence of air (flow rate, 8 mlg⁻¹min⁻¹) in a down flow tubular furnace. After calcination, the catalyst was cooled in presence of air and the catalyst was stored in an air tight container. The final calculated composition of the catalyst corresponds to V₂O₅ - 6.3 wt. %, K₂O - 0.6 wt. %, La₂O₃ - 0.5 wt. % with remaining part comprising δ-Al₂O₃.

### Comparative Example 3

### (Synthesis of Vanadium Oxide - K₂O Supported on Silica Modified Delta Alumina)

The silica modified alumina extrudates mainly in delta-alumina (δ-Al₂O₃) form were prepared using following procedure. Boehmite (G-250) material was extrudated using nitric acid as a peptizing agent. To boehmite powder (500 g) was added dropwise 5% nitric acid (370 ml) containing silica sol (9.25 ml of silica sol (AS-40) and the mixture was mixed for about 30 minutes. The obtained dough was then extruded using a lab extruder (Sunsai), the dies being 3.5 mm in diameter. The prepared wet extrudates were dried at 120 °C for 16 hours in air oven. The dried extrudates (160 g) were calcined at 950 °C for 5 hours in muffle furnace with heating rate 5 °C/min and air flow rate being 480±10 ml/min. The obtained extrudates (size: 3 mm diameter and 6-8 mm length) with surface area of 145 m²/g were used for catalyst preparation. The alumina extrudate support (about 100 g) was heat treated in an oven at 120 °C for 16 hours in the presence of air to remove moisture. The dried alumina extrudate support, after cooling to room temperature, was used for catalyst preparation by incipient wetness impregnation method. The catalyst was prepared by incipient wetness impregnation of the support with an aqueous solution of vanadyl oxalate, VOC₂O₄2H₂O (3.97 g) and potassium nitrate (0.386 g). The impregnation was carried out by contacting the impregnation solution (18 mL) with alumina extrudate support (27.93 g) at room temperature. The impregnated alumina support was then kept at room temperature for 12 hours and then dried at 120 °C for 16 hours. The dried sample was then calcined at 750 °C for 4 hours with heating rate 5 °C/min in the presence of air (flow rate, 8 ml g⁻¹min⁻¹) in a down flow tubular furnace. After calcination, the catalyst was cooled in presence of air and the catalyst was stored in an air tight container. The final calculated composition of the catalyst corresponds to V₂O₅ - 6.3 wt. %, K₂O - 0.6 wt. %, SiO₂ - 1 wt. % with remaining part comprising and δ-Al₂O₃.

### Example 4

### (Synthesis of Vanadium Oxide - Lanthanum oxide - K2O Supported on Silica Modified Delta Alumina)

The silica modified alumina extrudates mainly in delta-alumina (δ-Al₂O₃) form were prepared using following procedure. Boehmite (G-250) material was extrudated using nitric acid as a peptizing agent. To boehmite powder (500 g) was added dropwise 5% nitric acid (370 ml) containing silica sol (9.25 ml of silica sol (AS-40) and the mixture was mixed for about 30 minutes. The obtained dough was then extruded using a lab extruder (Sunsai), the dies being 3.5 mm in diameter. The prepared wet extrudates were dried at 120 °C for 16 hours in air oven. The dried extrudates (160 g) calcined at 950 °C for 5 hours in muffle furnace with heating rate 5 °C/min and air flow rate being 480±10 ml/min. The obtained extrudates (size: 3 mm diameter and 6-8 mm length) with surface area of 145 m²/g were used for catalyst preparation. The alumina extrudate support (about 100 g) was heat treated in an oven at 120 °C for 16 hours in the presence of air to remove moisture. The dried alumina extrudate support, after cooling to room temperature, was used for catalyst preparation by incipient wetness impregnation method. The catalyst was prepared by incipient wetness impregnation of the support with an aqueous solution of vanadyl oxalate, VOC₂O₄2H₂O (3.97 g), potassium nitrate (0.386 g) and lanthanum nitrate (0.399 g). The impregnation was carried out by contacting the impregnation solution (18 mL) with alumina extrudate support (27.93 g) at room temperature. The impregnated alumina support was then kept at room temperature for 12 hours and then dried at 120 °C for 16 hours. The dried sample was then calcined at 750 °C for 4 hours with heating rate 5 °C/min in the presence of air (flow rate, 8 ml g⁻¹min⁻¹) in a down flow tubular furnace. After calcination, the catalyst was cooled in presence of air and the catalyst was stored in an air tight container. The final calculated composition of the catalyst corresponds to V₂O₅ - 6.3 wt. %, K₂O - 0.6 wt. %, La₂O₃ - 0.5 wt %, SiO₂ - 1.0 wt. % with remaining part comprising δ-Al₂O₃.

### Example 5

### (Catalyst Testing and Aging)

The dehydrogenation activities of the catalysts were measured in a tubular fixed-bed quartz reactor. The details of catalyst loading and reactor were as follows: catalyst weight = 4.0 g, catalyst particle size = 0.4-0.5 mm, reactor ID = 16 mm, reactor OD = 19 mm. Isobutane (99.9 vol. %) was used as the feed. Quartz chips with size of 1-1.4 mm were loaded above the catalyst bed. Nitrogen purge separated dehydrogenation and catalyst regeneration/oxidation and reduction with hydrogen. The total feed flow in the dehydrogenation step was corresponds to GHSV = 600 mLh⁻¹g⁻¹. The reactor outlet gases were analyzed by online gas chromatograph (Agilent 6890, Agilent Scientific Instruments, USA) equipped with a flame ionization detector for hydrocarbon analysis and thermal conductivity detector for hydrogen analysis. The reactant and products flow rates were measured using Ritter type wet gas flow meter. The reactor was operated at atmospheric pressure and in a cyclic mode with the following steps:
1. Oxidation in air at 650 °C for 20 min.
2. Purge with nitrogen at 650 °C for 5 min.
3. Reduction with H₂ at 650 °C for 6 min.
4. Cooling with nitrogen from 650 °C to 585 °C with 20 min. hold at 585 °C.
5. Dehydrogenation of isobutane at 585 °C for 21 min.
6. GC analysis at 20th minute from the start of the isobutane feed.
7. Heating with nitrogen from 585 °C to 650 °C with 5 min. hold at 650 °C.
8. Steps 1-7 were repeated for 30 cycles before aging of the catalyst and 15 cycles after aging of the catalysts.

The catalyst aging protocol details are discussed below. Catalyst stability (for first two aging) evaluation was carried out by accelerated aging procedure in a cyclic mode of operation. The cycle included H₂-isobutane-air passing stages with suitable time intervals. The aging was carried out at 700 °C for 3 days. The other details are as follows: catalyst weight = 4.0 g, GHSV for all gases = 600 ml g⁻¹ h⁻¹, except for air 1200 ml g⁻¹ h⁻¹ for first aging and 1800 ml g⁻¹ h⁻¹ for second aging.
1. Oxidation in air for 20 min.
2. Purge with nitrogen for 5 min.
3. Reduction with H₂ for 6 min.
4. Purge with nitrogen for 5 min.
5. Iso-butane flow for 5 min.
6. Purge with nitrogen for 5 min.
7. Steps 1 to 6 were repeated for 75 cycles.

The third aging was carried out at 750 °C for 3 days (Comparative Example-3 and Example-4). The other details are as follows: catalyst weight = 4.0 g, GHSV for all gases = 600 mL g⁻¹ h⁻¹ except for air 1800 mL g⁻¹ h⁻¹.
1. Oxidation in air for 20 min.
2. Purge with nitrogen for 5 min.
3. Reduction with H₂ for 6 min.
4. Purge with nitrogen for 5 min.
5. Iso-butane flow for 5 min.
6. Purge with nitrogen for 5 min.
7. Steps 1 to 6 were repeated for 50 cycles.

The fourth aging was carried out at 800 °C for 2 days (Comparative Example-3 and Example-4). The other details are as follows: catalyst weight = 4.0 g, GHSV for all gases = 600 mL g⁻¹ h⁻¹ except for air 1800 mL g⁻¹ h⁻¹.
1. Oxidation in air for 20 min.
2. Purge with nitrogen for 5 min.
3. Reduction with H2 for 6 min.
4. Purge with nitrogen for 5 min.
5. Iso-butane flow for 3 min.
6. Purge with nitrogen for 5 min.
7. Steps 1 to 6 were repeated for 50 cycles.

The fifth aging was carried out at 820 °C for 2 days (Comparative Example-3 and Example-4). The other details are as follows: catalyst weight = 4.0 g, GHSV for all gases = 600 mL g⁻¹ h⁻¹ except for except for air 1800 mL g⁻¹ h⁻¹ and IB 400 mL g⁻¹ h⁻¹.
1. Oxidation in air for 20 min.
2. Purge with nitrogen for 5 min.
3. Reduction with H₂ for 6 min.
4. Purge with nitrogen for 5 min.
5. Iso-butane flow for 3 min.
6. Purge with nitrogen for 5 min.
7. Steps 1 to 6 were repeated for 50 cycles.

The isobutane dehydrogenation results are presented in FIGS. 2A (conversion), 2B (selectivity), and 2C (yield) for comparative example-1, comparative example-2, comparative example-3 and example 4. Catalysts of comparative example-1 and comparative example-2 delivered lower conversion after 1^{st} aging. Hence from 2^{nd} aging onwards, further aging and reaction was continued for catalysts of comparative example-3 and example 4 only. FIGS. 2A, 2B and 2C show a catalyst according to an example of the present invention. Example 4 shows better catalyst performance (e.g., conversion, selectivity and yield) compared to the comparative catalysts (comparative examples 1, 2 and 3).

Although embodiments of the present application and their advantages have been described in detail, it should be understood that various changes, substitutions and alterations can be made herein without departing from the spirit and scope of the embodiments as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the above disclosure, processes, machines, manufacture, compositions of matter, means, methods, or steps, presently existing or later to be developed that perform substantially the same function or achieve substantially the same result as the corresponding embodiments described herein can be utilized. Accordingly, the appended claims are intended to include within their scope such processes, machines, manufacture, compositions of matter, means, methods, or steps.

## Claims

1. A supported non-oxidative alkane dehydrogenation catalyst comprising:
a support comprising silica and alumina;
a vanadium oxide;
a rare earth metal oxide; and
an alkali metal oxide.

2. The supported non-oxidative alkane dehydrogenation catalyst of claim 1, wherein the rare earth metal oxide is a lanthanum oxide, cerium oxide, neodymium oxide, samarium oxide, or any combination thereof, preferably a lanthanum oxide and/or cerium oxide, more preferably a lanthanum oxide.

3. The supported non-oxidative alkane dehydrogenation catalyst of any one of claims 1 or 2, wherein the alkali metal oxide is a lithium oxide, sodium oxide, potassium oxide, rubidium oxide, or cesium oxide or any combination thereof, preferably a potassium oxide.

4. The supported non-oxidative alkane dehydrogenation catalyst of any one of claims 1 to 3, wherein the catalyst comprises 1 to 20 wt. % of the vanadium oxide represented as V₂O₅, 0.1 to 3 wt. % of the rare earth metal oxide, or 0.1 to 2 wt. % of the alkali metal oxide or any combination thereof, based on the total weight of the catalyst.

5. The supported non-oxidative alkane dehydrogenation catalyst of claim 4, wherein the rare earth metal oxide is a lanthanum oxide, and/or a cerium oxide, preferably a lanthanum oxide and/or wherein the alkali metal oxide is a potassium oxide.

6. The supported non-oxidative alkane dehydrogenation catalyst of any one of claims 1 to 4, wherein the amount of silica in the support is 0.1 to 5 wt. %, based on the total weight of the catalyst.

7. The supported non-oxidative alkane dehydrogenation catalyst of any one of claims 1 to 6, wherein the alumina in the catalyst support is a transition alumina, such as δ - alumina.

8. The supported non-oxidative alkane dehydrogenation catalyst of claim 1, comprising:
0.1 to 5 wt. % of silica and 71 wt. % to 98.7 wt.% of alumina in the support, based on the total weight of the catalyst;
1 to 20 wt. % of the vanadium oxide based on the total weight of the catalyst;
0.1 to 3 wt. % of the rare earth metal oxide based on the total weight of the catalyst; and
0.1 to 2 wt. % of the alkali metal oxide based on the total weight of the catalyst.

9. The supported non-oxidative alkane dehydrogenation catalyst of claim 8, wherein the alumina in the support comprises δ - alumina, the rare earth metal oxide is a lanthanum oxide, such as La₂O₃, and the alkali metal oxide is a potassium oxide, such as K₂O.

10. The supported non-oxidative alkane dehydrogenation catalyst of any one of claims 1 to 9, wherein the catalyst has a specific surface area of at least 50 m²/g, such as 50 m²/g to 250 m²/g.

11. A process for producing of an alkene, the process comprising contacting a feed stream comprising an alkane with a supported non-oxidative alkane dehydrogenation catalyst of any one of claims 1 to 10 under conditions sufficient to non-oxidatively dehydrogenate at least a portion of the alkane and produce a product stream comprising an alkene.

12. The process of claim 11, wherein the contacting conditions include a temperature of 400 °C to 800 °C and/or a pressure of 0.01 MPa to 0.5 MPa, and/or wherein the alkane is a C₂ to C₁₀ alkane, preferably a C₂ to C₄ alkane.

13. A process for producing a supported non-oxidative alkane dehydrogenation catalyst of any one of claims 1 to 10, the process comprising:
(a) impregnating a calcined support comprising alumina and silica with a vanadium precursor, a rare earth metal precursor and an alkali metal precursor to obtain an impregnated support;
(b) drying the impregnated support to obtain a dried impregnated support; and
(c) calcining the dried impregnated support to produce the supported non-oxidative alkane dehydrogenation catalyst.

14. The process of claim 13, wherein the process further comprises making the calcined support comprising:
(d) contacting an alumina precursor with a silica precursor under conditions suitable to obtain a shapeable support precursor;
(e) shaping the shapeable support precursor to obtain a wet shaped support precursor;
(f) drying the wet shaped support precursor to obtain a dried shaped support precursor; and
(g) calcining the dried shaped support precursor to obtain the calcined support.

15. The process of claim 13 or 14, wherein in step (c) the dried impregnated support is calcined at 500 to 850 °C and/or wherein shaping in step (e) comprises extruding the shapeable support precursor.
